# EUROPEAN PATENT SPECIFICATION

(11) **EP 3 956 460 B1**
(45) Date of publication and mention of the grant of the patent: **11.02.2026**
(21) Application number: 20746744.0
(22) Date of filing: 20.04.2020
(51) Int. Cl.: C12N 15/90, C12N 15/85

(54) **STABLE TARGETED INTEGRATION**
STABILE, GEZIELTE INTEGRATION
INTÉGRATION CIBLÉE STABLE

(30) Priority: 18.04.2019 US 201962835810 P
(43) Date of publication of application: 23.02.2022
(73) Proprietor: Sigma-Aldrich Co. LLC, St. Louis, MO 63103 (US)
(72) Inventor: BAHR, Scott, St. Louis, MO 63103 (US); JOHNS, Michael, St. Louis, MO 63103 (US); RAVELLETTE, James, St. Louis, MO 63103 (US); MASCARENHAS, Joaquina, St. Louis, MO 63103 (US); BORGSCHULTE, Trissa, St. Louis, MO 63103 (US)
(74) Representative: Haseltine Lake Kempner LLP
(86) International application number: PCT/US2020/028991
(87) International publication number: WO 2020/215077

(56) References cited:
- WO-A1-2018/148196
- WO-A1-2019/030373

## Description

### RELATED APPLICATION

The present application claims the benefit of priority of U.S. Provisional Patent Application No. 62/835,810, filing date April 18, 2019.

### FIELD

The present disclosure relates to the stable integration of exogenous sequences into genomic loci where the exogenous sequences can function predictably and reliably.

### BACKGROUND

Traditional cell line engineering approaches have used methods to randomly insert transgenes into the genome of the host cell. Such engineering approaches have led to the development of highly productive cell lines for recombinant therapeutic protein expression. However, such integration methods have led to unstable cell lines and clonal populations that are markedly diverse for expression of the same molecule in terms of expression level and protein heterogeneity. To circumvent these issues, site-specific targeted integration of transgenes is desired for recombinant therapeutic protein expression.

The key to successful site-specific targeted integration of transgenes depends on a suitable genomic location (i.e., a "safe harbor") to target for integration. This location must be amenable to transgene or exogenous sequence insertion, allow for predictable and stable expression of the transgene, and must not interfere with cellular growth and function. A suitable site at the AAVS1 locus has been identified for human- derived cell lines, but viable sites in many cells used for therapeutic protein production have not been identified. Thus, there is a need to identify and verify suitable genomic locations in Chinese hamster ovary (CHO) and other cells for the successful integration of therapeutic protein cassettes or other exogenous sequences. WO 2018/148196A describes methods for integrating exogenous sequences in genomic loci. WO 2019/030373A describes integration sites in CHO cells.

### BRIEF DESCRIPTION OF THE FIGURES

FIG. **1A** shows RMCE compatible cassettes used in hot spot identification and validation approach 1.
FIG. **1B** shows RMCE compatible cassettes used in hot spot identification and validation approach 2.
FIG. **2** shows flow cytometry analysis of IgG bound to cell surfaces. Left: D145 clone with GFP landing pad. Middle: Random integration of IgG payload. Right: Targeted integration of IgG payload using Cre recombinase.
FIG. **3** shows positive junction PCR in pools containing the targeted integration product but not the random integration pool.
FIG. **4** shows the ZFN target at the MP 7 TI site and a Cell assay displaying positive ZFN cutting activity.
FIG. **5** shows the ZFN target at the MP 58 TI site and a Cell assay displaying positive ZFN cutting activity.
FIG. **6** shows monoclonal antibody production from the MP 58 TI site.

### SUMMARY

The present invention provides a method for stable integration of at least one exogenous sequence into genomic DNA of a Chinese hamster ovary (CHO) cell, the method comprises integrating the at least one exogenous sequence into a site within the region bp 1,090,000 to bp 1,127,00 of genomic sequence NCBI Reference Sequence NW_003613934.1. Generally described herein is a method for stable integration of at least one exogenous sequence into genomic DNA of a cell. The method comprises integrating the at least one exogenous sequence into a site within a genomic sequence chosen from NCBI Reference Sequences NW_003613934.1, NW_003614159.1, NW_003613732.1, or homolog thereof.

Generally described herein is a method for preparing a cell comprising an exogenous sequence integrated into genomic DNA. The method comprises (a) introducing into the cell (i) a targeting endonuclease or nucleic acid encoding the targeting endonuclease, wherein the targeting endonuclease is targeted to a target site within a genomic sequence chosen from NCBI Reference Sequences NW_003613934.1, NW_003614159.1, NW_003613732.1, or homolog thereof and (ii) a donor polynucleotide comprising the exogenous sequence; and (b) maintaining the cell under conditions such that the exogenous sequence is integrated into the target site of the genomic sequence.

Some embodiments generally described herein provide a host cell, preferably a host cell line, comprising at least one exogenous sequence integrated into genomic DNA in at least one predefined target site suitable for stable integration. Suitable predefined target sites are preferably located within a genomic sequence chosen from the group consisting of NCBI Reference Sequences NW_003613934.1, NW_003614159.1, NW_003613732.1, or homolog thereof.

Other aspects and iterations of the disclosure are described in more detail below.

### DETAILED DESCRIPTION

The present disclosure provides genomic loci for stable integration of exogenous sequences and methods for integrating exogenous sequences into these genomic loci. The exogenous sequences are stably integrated into these genomic loci where they can function predictably and reliably. The genomic loci, therefore, can be termed "safe harbors." The integrated sequence remains in the genomic locus and is not excised or altered in any manner. For example, the integrated sequence and adjacent sequences are not subject to gene silencing or position effects. Additionally, the integrated exogenous sequence does not affect the function of genes or other chromosomal sequences in the cell, i.e., global or local gene expression is not altered, there are no cell abnormalities or deficits, there is no position mutagenesis or other side effects, etc.. Moreover, when the exogenous sequence encodes a protein or RNA molecule, expression of the exogenous sequence is stable, efficient, consistent, and predictable.

### (I) Genomic Loci for Stable Integration

The present disclosure generally provides mammalian genomic loci in which exogenous sequences can integrate and function predictably and reliably. The genomic locus suitable for stable integration are located within genomic sequences chosen from NCBI Reference Sequences (RefSeq) NW_003613934.1 (CriGri_1.0 Scaffold979), NW_003614159.1(CriGri_1.0 Scaffold3466), NW_003613732.1 (CriGri_1.0 Scaffold1721), or homolog thereof. The listed RefSeqs are contigs/scaffolds from the genome of Chinese hamster, but homologous sequences are present in other mammalian genomes (e.g., human, mouse, rat, monkey, canine, bovine, and so forth) and can be used for stable integration in these mammalian cells.

A preferred locus is the region bp 1,090,000 to bp 1,127,000 of NW_003613934.1 (CriGri_1.0 Scaffold979), which is flanked by the genes Transfer RNA lysine (Trnak) on the 5' and Peroxisomal NADH pyrophosphatase enzyme (NUDT12) on the 3'. Another preferred locus is the region bp 365,000 to bp 395,000 of NW_003614159.1(CriGri_1.0 Scaffold3466), which is flanked by the genes RAB6-interacting golgin (Gorab) on the 5' and Paired mesoderm homeobox protein 1 (Prrx1) on the 3'. Another preferred locus is bp 1,935,000 to bp 1,985,000 of NW_003613732.1 (CriGri_1.0 Scaffold1721), which is flanked by the genes Alpha-mannosidase 2 (Man2a1) on the 5' and Transmembrane protein 232 (Tmem 232) on the 3'.

A more precise insertion position for each preferred locus was determined using the CHOK1S-HZDv1 assembly (GenBank Assembly Accession: GCA_900186095.1) as a reference genome. Site D145 is an insertion at (Scaffold:Position) 0: 212,357,115. Site MP 7 is an insertion at 0: 217,336,436 - 217,336,341. Site MP 58 is an insertion at 1: 28,570,918 - 28,570,919.

### II. Methods for Stable Integration of Exogenous Sequences

The present disclosure generally provides methods for stable integration of one or more exogenous sequences into genomic DNA of a cell, wherein the method comprises integrating the at least one exogenous sequence into a site within a genomic sequence chosen from NCBI Reference Sequences NW_003613934.1, NW_003614159.1, NW_003613732.1, or homolog thereof. The integrated sequence does not adversely affect the cell and the function of the integrated sequence is predictable, consistent, and reproducible.

In particular, the method comprises introducing into the cell (i) a targeting endonuclease that is targeted to a target site within a genomic sequence chosen from NCBI Reference Sequences NW_003613934.1, NW_003614159.1, NW_003613732.1, or homolog thereof and (ii) a donor polynucleotide comprising the at least one exogenous sequence, and maintaining the cell under conditions such that the at least one exogenous sequence is integrated into the genome of the cell.

### (a) Exogenous sequence

As used herein, an "exogenous" sequence refers to a nucleotide sequence that is not native to the cell, or a nucleotide sequence whose native location is in a different location in the genome of the cell.

In some embodiments, the exogenous sequence encodes a protein. The encoded protein can be a recombinant protein, a therapeutic protein, or an industrial protein. Non-limiting examples of suitable proteins include antibodies, antibody fragments, monoclonal antibodies , humanized antibodies, humanized monoclonal antibodies, chimeric antibodies, IgG molecules, IgG heavy chains, IgG light chains, IgA molecules, IgD molecules, IgE molecules, IgM molecules, vaccines, growth factors, cytokines, interferons, interleukins, hormone, clotting (or coagulation) factors, blood components, enzymes, nutraceutical proteins, functional fragments or variants of any of the forgoing, or fusion proteins comprising any of the foregoing proteins and/or functional fragments or variants thereof.

In other embodiments, the exogenous sequence encodes a RNA molecule, e.g., a non-coding RNA (ncRNA). Non-limiting examples of ncRNA include micro RNA (miRNA), small interfering RNA (siRNA), guide RNA (gRNA), long noncoding RNA (IncRNA), long intergenic non-coding RNA (lincRNA), Piwi-interacting RNA (piRNA), trans-acting RNA (rasiRNA), ribosomal RNA (rRNA), transfer RNA (tRNA), mitochondrial tRNA (MT-tRNA), small nuclear RNA (snRNA), small nucleolar RNA (snoRNA), SmY RNA, Y RNA, spliced leader RNA (SL RNA), telomerase RNA component (TERC), fragments thereof, or combinations thereof. In particular embodiments, the exogenous sequence can encode a miRNA, a siRNA, or a gRNA.

In still other embodiments, the exogenous sequence comprises at least one recognition sequence for at least one polynucleotide modification enzyme. Stated another way, the exogenous sequence comprises a "landing pad," wherein the landing pad can be used for subsequent targeted integration of exogenous sequences. The recognition sequence for the at least one polynucleotide modification enzyme generally does not exist endogenously in the genome of the cell. Selection of a recognition sequence that does not exist endogenously in the cell may increase the rate of targeted integration and/or reduce potential off-target integration. The polynucleotide modification enzyme can be a site-specific recombinase or a targeting endonuclease. Non-limiting examples of site-specific recombinases may include Bxb1 integrase, Cre recombinase, FLP recombinase, gamma delta resolvase, lambda integrase, phi C31 integrase, R4 integrase, Tn3 resolvase, and TP901-1 recombinase. Site-specific recombinases recognize specific recognition sequences (or recognition sites), which are well known in the art. For example, Cre recombinases recognize LoxP sites and FLP recombinases recognize FRT sites. Contemplated targeting endonucleases include zinc finger nucleases (ZFNs), clustered regularly interspersed short palindromic repeats (CRISPR)/CRISPR-associated (Cas) (CRISPR/Cas) nuclease systems, CRISPR/Cas dual nickase systems, transcription activator-like effector nucleases (TALENs), meganucleases, or fusion proteins comprising programmable DNA-binding domains and nuclease domains. Each of these targeting endonucleases is further described below in section (II)(c).

Multiple recognition sequences may be present in a single landing pad, allowing the landing pad to be targeted sequentially by two or more polynucleotide modification enzymes such that two or more exogenous sequences can be inserted. Alternatively, the presence of multiple recognition sequences in the landing pad, allows multiple copies of the same exogenous sequence to be inserted into the landing pad. When two exogenous sequences are targeted to a single landing pad, the landing pad includes a first recognition sequence for a first polynucleotide modification enzyme (such as a first ZFN pair), and a second recognition sequence for a second polynucleotide enzyme (such as a second ZFN pair). Alternatively, or additionally, individual landing pads comprising one or more recognition sequences may be integrated at multiple locations within a cell's genome to permit multi-copy integration of exogenous sequences comprising recombinant protein expression constructs. Increased protein expression may be observed in cells transformed with multiple copies of an exogenous sequence comprising an expression construct. Alternatively, multiple protein products may be expressed simultaneously when multiple unique exogenous sequences comprising different expression cassettes are inserted, whether in the same or a different landing pad. For example, the exogenous landing pad can comprise at least one, at least two, at least three, at least four, at least five, at least six, at least seven, at least eight, at least nine, or at least ten or more recognition sequences. In embodiments comprising more than one recognition sequence, the recognition sequences may be unique from one another *(*i.e., recognized by different polynucleotide modification enzymes), the same repeated sequence, or a combination of repeated and unique sequences.

One of ordinary skill in the art will readily understand that the exogenous sequence can include additional sequences. For example, protein and RNA coding sequences can be operably linked to promoter control sequences for expression in the cell of interest. In embodiments in which the exogenous sequence encodes a protein, the exogenous sequence can be operably linked to a promoter sequence that is recognized by RNA polymerase II (Pol II). The Pol II promoter control sequence can be constitutive, regulated, or tissue-specific. Suitable constitutive Pol II promoter control sequences include, but are not limited to, cytomegalovirus immediate early promoter (CMV), simian virus (SV40) promoter, adenovirus major late promoter, Rous sarcoma virus (RSV) promoter, mouse mammary tumor virus (MMTV) promoter, phosphoglycerate kinase (PGK) promoter, elongation factor (ED1)-alpha promoter, ubiquitin promoters, actin promoters, tubulin promoters, immunoglobulin promoters, fragments thereof, or combinations of any of the foregoing. Examples of suitable Pol II regulated promoter control sequences include without limit those regulated by heat shock, metals, steroids, antibiotics, or alcohol. Non-limiting examples of Pol II tissue-specific promoters include B29 promoter, CD14 promoter, CD43 promoter, CD45 promoter, CD68 promoter, desmin promoter, elastase-1 promoter, endoglin promoter, fibronectin promoter, Flt-1 promoter, GFAP promoter, GPllb promoter, ICAM-2 promoter, INF-β promoter, Mb promoter, Nphsl promoter, OG-2 promoter, SP-B promoter, SYN1 promoter, and WASP promoter. The promoter control sequence can be wild type or it can be modified for more efficient or efficacious expression. The protein coding sequence also can be linked to polyadenylation signals (*e.g*., SV40 polyA signal, bovine growth hormone (BGH) polyA signal, etc.) and/or transcriptional termination sequences.

In embodiments in which the exogenous sequence encodes RNA, the exogenous sequence can be operably linked to a promoter control sequence that is recognized by RNA polymerase III (Pol III). Examples of suitable Pol III promoters include, but are not limited to, mammalian U6, U3, H1, and 7SL RNA promoters. The RNA-coding exogenous sequence also can be linked to transcriptional termination sequences.

In additional embodiments, the exogenous sequence can be linked to sequence encoding hypoxanthine-guanine phosphoribosyltransferase (HPRT), dihydrofolate reductase (DHFR), and/or glutamine synthetase (GS), such that HPRT, DHFR, and/or GS may be used as an amplifiable selectable marker. The exogenous sequence also can be linked to sequence encoding at least one antibiotic resistance gene and/or sequence encoding reporter proteins such as fluorescent proteins. Non limiting examples of antibiotic resistance genes include those coding resistance for blasticidin, G418 (Geneticin^{®}), hydromycin B, puromycin, and phleomycin D1 (Zeocin^{™}). Suitable fluorescent proteins include without limit green fluorescent proteins (*e.g*., GFP, GFP-2, tagGFP, turboGFP, EGFP, Emerald, Azami Green, Monomeric Azami Green, CopGFP, AceGFP, ZsGreen1), yellow fluorescent proteins (*e.g*., YFP, EYFP, Citrine, Venus, YPet, PhiYFP, ZsYellow1), blue fluorescent proteins (*e.g*., BFP, EBFP, EBFP2, Azurite, mKalama1, GFPuv, Sapphire, T-sapphire), cyan fluorescent proteins (*e.g*., ECFP, Cerulean, CyPet, AmCyan1, Midoriishi-Cyan), red fluorescent proteins (*e.g*., mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), and orange fluorescent proteins (*e.g*., mOrange, mKO, Kusabira-Orange, Monomeric Kusabira-Orange, mTangerine, tdTomato) or any other suitable fluorescent protein.

In some preferred embodiments, the landing pad is configured to facilitate Recombinase Mediated Cassette Exchange (RMCE). In such embodiments the landing pad comprises a sequence encoding a selectable marker and a sequence encoding a reporter protein, and a first and second recognition sequence for a polynucleotide modification enzyme. These elements are configured such that the sequence encoding a selectable marker and the sequence encoding a reporter protein are between the first and second recognition sequence for a polynucleotide modification enzyme. In a particularly preferred embodiment, the sequence encoding a reporter protein encodes a green fluorescent protein, such as GFP or turboGFP; the sequence encoding a selectable marker encodes puromycin; and the first and second recognition sequences are Lox sequences. Suitable Lox sequences include Lox71, Lox2272, Lox66 and Lox66/71.

### (b) Donor polynucleotide comprising exogenous sequence

The method generally described herein comprises introducing a donor polynucleotide comprising the exogenous sequence(s) into the cell. In some embodiments, the exogenous sequence in the donor polynucleotide can be flanked by sequences having substantial sequence identity to sequences flanking the target site in the genomic sequence. For example, the exogenous sequence can be flanked by an upstream sequence and a downstream sequence, wherein the upstream and downstream sequences have substantial sequence identity with sequence on either side of the target site in the genomic sequence. The upstream sequence, as used herein, refers to a nucleic acid sequence that shares substantial sequence identity with the genomic sequence immediately upstream of the targeted site. Similarly, the downstream sequence refers to a nucleic acid sequence that shares substantial sequence identity with the genomic sequence immediately downstream of the targeted site. The upstream and downstream sequences in the donor polynucleotide comprising the exogenous sequence are selected to promote recombination between the targeted genomic sequence and the donor polynucleotide (comprising the exogenous sequence).

As used herein, the phrase "substantial sequence identity" refers to sequences having at least about 75% sequence identity. Thus, the upstream and downstream sequences in the donor polynucleotide comprising the exogenous sequence may have about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with chromosomal sequence adjacent (i.e., upstream or downstream) to the target site in the genomic sequence. In specific embodiments, the upstream and downstream sequences in the donor polynucleotide comprising the exogenous sequence have about 95% or 100% sequence identity with chromosomal sequences adjacent to the target site in the genomic sequence. An upstream or downstream flanking sequence may comprise from about 10 bp to about 2500 bp. In one embodiment, an upstream or downstream sequence may comprise about 20, 30, 40, 50, 60, 70, 80, 90, 100, 125, 150, 175, 200, 300, 400, 500, 600, 700, 800, 900, 1000, 1100, 1200, 1300, 1400, 1500, 1600, 1700, 1800, 1900, or 2000 bp. An exemplary upstream or downstream flanking sequence may comprise from about 20 to about 200 bp, from 25 to about 100 bp, or from about 40 bp to about 60 bp. In certain embodiments, the upstream or downstream flanking sequence may comprise from about 200 to about 500 bp.

In other embodiments, the exogenous sequence in the donor polynucleotide can be flanked by sequences that are recognized by the targeting endonuclease. For example, the exogenous sequence can be flanked by an upstream sequence and a downstream sequence, wherein the upstream and downstream sequences comprise the recognition sequence of the targeting endonuclease. Thus, the targeting endonuclease can introduce a double stranded break at the targeted site in the genomic sequence and double stranded breaks in the donor polynucleotide such that the exogenous sequence is released from the rest of the donor polynucleotide, wherein exogenous sequence can be directly ligated with the cleaved genomic sequence leading to integration of the exogenous sequence into the genome of the cell.

The donor polynucleotide comprising the exogenous sequence can be single stranded or double stranded, linear, or circular. Generally, the donor polynucleotide is DNA. The donor polynucleotide can be a vector. Suitable vectors include plasmid vectors, phagemids, cosmids, artificial/mini-chromosomes, transposons, and viral vectors. Non-limiting examples of suitable plasmid vectors include pUC, pBR322, pET, pBluescript, and variants thereof. The donor polynucleotide can comprise additional control sequences (*e.g*., promoter sequences, enhancer sequences, Kozak sequences, polyadenylation sequences, transcriptional termination sequences, *etc.*)*,* origins of replication, selectable marker sequences (*e.g*., antibiotic resistance genes), and the like. Additional information can be found in "Current Protocols in Molecular Biology" Ausubel et al., John Wiley & Sons, New York, 2003 or "Molecular Cloning: A Laboratory Manual" Sambrook & Russell, Cold Spring Harbor Press, Cold Spring Harbor, NY, 3rd edition, 2001.

### (c) Targeting endonucleases

The method generally described herein also comprises introducing a targeting endonuclease or nucleic acid encoding a targeting endonuclease into the cell. A targeting endonuclease comprises a DNA-binding domain and a nuclease domain. The DNA binding domain of the targeting endonuclease is programmable, meaning that it can be designed or engineered to recognize and bind different DNA sequences. In some embodiments, the DNA binding is mediated by interactions between the DNA binding domain of the targeting endonuclease and the target DNA. Thus, the DNA-binding domain can be programed to bind a DNA sequence of interest by protein engineering. In other embodiments, DNA-binding is mediated by a guide RNA that interacts with the DNA-binding domain of the targeting endonuclease and the target DNA. In such instances, the DNA-binding domain can be targeted to a DNA sequence of interest by designing the appropriate guide RNA.

Suitable targeting endonuclease include zinc finger nucleases, clustered regularly interspersed short palindromic repeats (CRISPR)/CRISPR-associated (Cas) (CRISPR/Cas) nuclease systems, CRISPR/Cas nickase systems, transcription activator-like effector nucleases, meganucleases, or fusion proteins comprising programmable DNA-binding domains and nuclease domains. The targeting endonuclease can comprise wild-type or naturally-occurring DNA-binding and/or nuclease domains, modified versions of naturally-occurring DNA-binding and/or nuclease domains, synthetic or artificial DNA-binding and/or nuclease domains, or combinations thereof.

### (i) Zinc finger nucleases

In some embodiments, the targeting endonuclease can be a zinc finger nuclease (ZFN). A ZFN comprise a DNA-binding zinc finger region and a nuclease domain. The zinc finger region can comprise from about two to seven zinc fingers, for example, about four to six zinc fingers, wherein each zinc finger binds three nucleotides, and wherein the zinc fingers can be linked together using suitable linker sequences. The zinc finger region can be engineered to recognize and bind to any DNA sequence. See, for example, Beerli et al. (2002) Nat. Biotechnol. 20:135-141; Pabo et al. (2001) Ann. Rev. Biochem. 70:313-340; Isalan et al. (2001) Nat. Biotechnol. 19:656-660; Segal et al. (2001) Curr. Opin. Biotechnol. 12:632-637; Choo et al. (2000) Curr. Opin. Struct. Biol. 10:411-416; Zhang et al. (2000) J. Biol. Chem. 275(43):33850-33860; Doyon et al. (2008) Nat. Biotechnol. 26:702-708; and Santiago et al. (2008) Proc. Natl. Acad. Sci. USA 105:5809-5814. Publically available web-based tools for identifying potential target sites in DNA sequences as well as designing zinc finger binding domains are known in the art.

A ZFN also comprises a nuclease domain, which can be obtained from any endonuclease or exonuclease. Non-limiting examples of endonucleases from which a nuclease domain can be derived include, but are not limited to, restriction endonucleases and homing endonucleases. A cleavage domain also may be derived from an enzyme or portion thereof that requires dimerization for cleavage activity. Two zinc finger nucleases may be required for cleavage, as each nuclease comprises a monomer of the active enzyme dimer. When two cleavage monomers are used to form an active enzyme dimer, the recognition sites for the two zinc finger nucleases are generally disposed such that binding of the two zinc finger nucleases to their respective recognition sites places the cleavage monomers in a spatial orientation to each other that allows the cleavage monomers to form an active enzyme dimer, e.g., by dimerizing. As a result, the near edges of the recognition sites may be separated by about 5 to about 18 nucleotides. For instance, the near edges may be separated by about 5, 6, 7, 8, 9, 10, 11, 12, 13, 14, 15, 16, 17 or 18 nucleotides.

In some embodiments, the nuclease domain can be derived from a type II-S restriction endonuclease. Type II-S endonucleases cleave DNA at sites that are typically several base pairs away from the recognition/binding site and, as such, have separable binding and cleavage domains. These enzymes generally are monomers that transiently associate to form dimers to cleave each strand of DNA at staggered locations. Non-limiting examples of suitable type II-S endonucleases include Bfil, Bpml, Bsal, Bsgl, BsmBI, Bsml, BspMI, Fokl, Mboll, and Sapl. In some embodiments, the nuclease domain can be a Fokl nuclease domain or a derivative thereof. The type II-S nuclease domain can be modified to facilitate dimerization of two different nuclease domains. For example, the cleavage domain of Fokl can be modified by mutating certain amino acid residues. By way of non-limiting example, amino acid residues at positions 446, 447, 479, 483, 484, 486, 487, 490, 491, 496, 498, 499, 500, 531, 534, 537, and 538 of Fokl nuclease domains are targets for modification. For example, one modified Fokl domain can comprise Q486E, I499L, and/or N496D mutations, and the other modified Fokl domain can comprise E490K, I538K, and/or H537R mutations.

The ZFN can further comprise at least one nuclear localization signal, cell-penetrating domain, and/or marker domain, which are described below in section (ll)(c)(vii).

### (ii) CRISPR/Cas nuclease systems

In other embodiments, the targeting endonuclease can be a RNA-guided CRISPR/Cas nuclease system, which introduces a double-stranded break in the DNA. The CRISPR/Cas nuclease system comprises a CRISPR/Cas nuclease and a guide RNA.

The CRISPR/Cas nuclease can be derived from a type I (*i.e.,* IA, IB, IC, ID, IE, or IF), type II (*i.e.,* IIA, IIB, or IIC), type III (i., IIIA or IIIB), or type V CRISPR system, which are present in various bacteria and archaea. The CRISPR/Cas system can be from *Streptococcus sp.* (*e.g., Streptococcus pyogenes*), *Campylobacter sp.* (*e.g., Campylobacter jejuni*)*, Francisella sp.* (*e.g., Francisella novicida*)*, Acaryochloris sp., Acetohalobium sp., Acidaminococcus sp., Acidithiobacillus sp., Alicyclobacillus sp., Allochromatium sp., Ammonifex sp., Anabaena sp., Arthrospira sp., Bacillus sp., Burkholderiales sp., Caldicelulosiruptor sp., Candidatus sp., Clostridium sp., Crocosphaera sp., Cyanothece sp., Exiguobacterium sp., Finegoldia sp., Ktedonobacter sp., Lactobacillus sp., Lyngbya sp., Mannobacter sp., Methanohalobium sp., Microscilla sp., Microcoleus sp., Microcystis sp., Natranaerobius sp., Neisseria sp., Nitrosococcus sp., Nocardiopsis sp., Nodularia sp., Nostoc sp., Oscillatoria sp., Polaromonas sp., Pelotomaculum sp., Pseudoalteromonas sp., Petrotoga sp., Prevotella sp., Staphylococcus sp., Streptomyces sp., Streptosporangium sp., Synechococcus sp.,* or *Thermosipho sp.*

Non-limiting examples of suitable CRISPR nuclease include Cas proteins, Cpf proteins, Cmr proteins, Csa proteins, Csb proteins, Csc proteins, Cse proteins, Csf proteins, Csm proteins, Csn proteins, Csx proteins, Csy proteins, Csz proteins, and derivatives or variants thereof. In specific embodiments, the CRISPR/Cas nuclease can be a type II Cas9 protein, a type V Cpf1 protein, or a derivative thereof. In some embodiments, the CRISPR/Cas nuclease can be *Streptococcus pyogenes* Cas9 (SpCas9) or *Streptococcus thermophilus* Cas9 (StCas9). In other embodiments, the CRISPR/Cas nuclease can be *Campylobacter jejuni* Cas9 (CjCas9). In alternate embodiments, the CRISPR/Cas nuclease can be *Francisella novicida* Cas9 (FnCas9). In yet other embodiments, the CRISPR/Cas nuclease can be *Francisella novicida* Cpf1 (FnCpf1).

In general, the CRISPR/Cas nuclease comprises a RNA recognition and/or RNA binding domain, which interacts with the guide RNA. The CRISPR/Cas nuclease also comprises at least one nuclease domain having endonuclease activity. For example, a Cas9 protein can comprise a RuvC-like nuclease domain and a HNH-like nuclease domain, and a Cpf1 protein can comprise a RuvC-like domain. CRISPR/Cas nucleases can also comprise DNA binding domains, helicase domains, RNase domains, protein-protein interaction domains, dimerization domains, as well as other domains.

The CRISPR/Cas nuclease can further comprise at least one nuclear localization signal, cell-penetrating domain, and/or marker domain, which are described below in section (ll)(c)(vii).

The CRISPR/Cas nuclease system also comprises a guide RNA (gRNA). The guide RNA interacts with the CRISPR/Cas nuclease to guide it to a target site in the genomic sequence. The target site has no sequence limitation except that the sequence is bordered by a protospacer adjacent motif (PAM). For example, PAM sequences for Cas9 include 3'-NGG, 3'-NGGNG, 3'-NNAGAAW, and 3'-ACAY and PAM sequences for Cpf1 include 5'-TTN (wherein N is defined as any nucleotide, W is defined as either A or T, and Y is defined an either C or T). Each gRNA comprises a sequence that is complementary to the target sequence (*e.g*., a Cas9 gRNA can comprise GN₁₇₋₂₀GG). The gRNA can also comprise a scaffold sequence that forms a stem loop structure and a single-stranded region. The scaffold region can be the same in every gRNA. In some embodiments, the gRNA can be a single molecule (*i.e*., sgRNA). In other embodiments, the gRNA can be two separate molecules (*i.e*., crRNA and tracrRNA).

### (iii) CRISPR/Cas nickase systems

In other embodiments, the targeting endonuclease can be a paired CRISPR/Cas nickase system. CRISPR/Cas nickase systems are similar to the CRISPR/Cas nuclease systems described above except that the CRISPR/Cas nuclease is modified to cleave only one strand of DNA. Thus, a single CRISPR/Cas nickase system creates a single-stranded break or nick in double-stranded DNA. Alternatively, a paired CRISPR/Cas nickase system (or dual nickase system) comprising a pair of offset gRNAs can create a double-stranded break in the DNA by generating single-stranded breaks on opposite strands of the DNA.

A CRISPR/Cas nuclease can be converted to a nickase by one or more mutations and/or deletions. For example, a Cas9 nickase can comprise one or more mutations in one of the nuclease domains, wherein the one or more mutations can be D10A, E762A, and/or D986A in the RuvC-like domain or the one or more mutations can be H840A, N854A and/or N863A in the HNH-like domain.

### (iv) Transcription activator-like effector nucleases

In alternate embodiments, the targeting endonuclease can be a transcription activator-like effector nuclease (TALEN). TALENs comprise a DNA-binding domain composed of highly conserved repeats derived from transcription activator-like effectors (TALEs) that is linked to a nuclease domain. TALEs are proteins secreted by plant pathogen *Xanthomonas* to alter transcription of genes in host plant cells (Bai et al., 2000, Mol. Plant Microbe Interact., 13(12):1322-9) TALE repeat arrays can be engineered via modular protein design to target any DNA sequence of interest. The nuclease domain of TALENs can be any nuclease domain as described above in section (II)(c)(i). In specific embodiments, the nuclease domain is derived from Fokl (Sanjana et al., 2012, Nat Protoc, 7(1):171-192).

The TALEN can also comprise at least one nuclear localization signal, cell-penetrating domain, and/or marker domain, which are described below in section (ll)(c)(vii).

### (v) Meganucleases or rare-cutting endonucleases

In still other embodiments, the targeting endonuclease can be a meganuclease or derivative thereof. Meganucleases are endodeoxyribonucleases characterized by long recognition sequences, i.e., the recognition sequence generally ranges from about 12 base pairs to about 45 base pairs. As a consequence of this requirement, the recognition sequence generally occurs only once in any given genome. Among meganucleases, the family of homing endonucleases named LAGLIDADG has become a valuable tool for the study of genomes and genome engineering (Arnould et al., 2011, Protein Engineering, Design & Selection, 24(1-2):27-31). Other suitable meganucleases include I-Crel, I-Dmol, I-Scel, I-Tevl, and variants thereof. A meganuclease can be targeted to a specific chromosomal sequence by modifying its recognition sequence using techniques well known to those skilled in the art.

In alternate embodiments, the targeting endonuclease can be a rare-cutting endonuclease or derivative thereof. Rare-cutting endonucleases are site-specific endonucleases whose recognition sequence occurs rarely in a genome, preferably only once in a genome. The rare-cutting endonuclease may recognize a 7-nucleotide sequence, an 8-nucleotide sequence, or longer recognition sequence. Non-limiting examples of rare-cutting endonucleases include Ascl, AsiSI, Fsel Notl, Pacl, and Sbfl.

The meganuclease or rare-cutting endonuclease can also comprise at least one nuclear localization signal, cell-penetrating domain, and or marker domain, which are described below in section (II)(c)(vii).

### (vi) Fusion proteins comprising nuclease domains

In yet additional embodiments, the targeting endonuclease can be a fusion protein comprising a nuclease domain and a programmable DNA-binding domain. The nuclease domain can be any of those described above in section (II)(c)(i), a nuclease domain derived from a CRISPR/Cas nuclease (*e.g*., RuvC-like or HNH-like nuclease domains of Cas9, or the nuclease domain of Cpf1), or a nuclease domain derived from a meganuclease or rare-cutting endonuclease.

The programmable DNA-binding domain of the fusion protein can be derived from a targeting endonuclease (*i.e*., CRISPR/CAS nuclease or meganuclease) that is modified to lack all nuclease activity (*i.e*., is catalytically inactive). Alternatively, the programmable DNA-binding domain of the fusion protein can be a programmable DNA-binding protein such as, e.g., a zinc finger protein or a TALE.

In some embodiments, the programmable DNA-binding domain can be a catalytically inactive CRISPR/Cas nuclease in which the nuclease activity was eliminated by mutation and/or deletion. For example, the catalytically inactive CRISPR/Cas protein can be a catalytically inactive (dead) Cas9 (dCas9) in which the RuvC-like domain comprises a D10A, E762A, and/or D986A mutation and the HNH-like domain comprises a H840A, N854A and/or N863A mutation. Alternatively, the catalytically inactive CRISPR/Cas protein can be a catalytically inactive (dead) Cpf1 protein comprising comparable mutations in the nuclease domain. In still other embodiments, the programmable DNA-binding domain can be a catalytically inactive meganuclease in which nuclease activity was eliminated by mutation and/or deletion, e.g., the catalytically inactive meganuclease can comprise a C-terminal truncation.

The fusion protein comprising a nuclease domain can also comprise at least one nuclear localization signal, cell-penetrating domain, and/or marker domain, which are described below in section (ll)(c)(vii).

### (vii) Optional additional domains

The targeting endonuclease can further comprise additional domains. For example, the targeting endonuclease can further comprise at least one nuclear localization signal, at least one cell-penetrating domain, and/or at least one marker domain.

In certain embodiments, the targeting endonuclease can comprise at least one NLS. In general, an NLS comprises a stretch of basic amino acids. Nuclear localization signals are known in the art (see, *e.g*., Lange et al., J. Biol. Chem., 2007, 282:5101-5105). For example, in one embodiment, the NLS can be a monopartite sequence, such as PKKKRKV (SEQ ID NO:1) or PKKKRRV (SEQ ID NO:2). In another embodiment, the NLS can be a bipartite sequence, such as KRPAATKKAGQAKKKK (SEQ ID NO:3).

In other embodiments, the targeting endonuclease can comprise at least one cell-penetrating domain. In one embodiment, the cell-penetrating domain can be a cell-penetrating peptide sequence derived from the HIV-1 TAT protein. As an example, the TAT cell-penetrating sequence can be GRKKRRQRRRPPQPKKKRKV (SEQ ID NO:4). In another embodiment, the cell-penetrating domain can be TLM (PLSSIFSRIGDPPKKKRKV; SEQ ID NO:5), a cell-penetrating peptide sequence derived from the human hepatitis B virus. In still another embodiment, the cell-penetrating domain can be MPG (GALFLGWLGAAGSTMGAPKKKRKV; SEQ ID NO:6 or GALFLGFLGAAGSTMGAWSQPKKKRKV; SEQ ID NO:7). In additional embodiments, the cell-penetrating domain can be Pep-1 (KETWWETWWTEWSQPKKKRKV; SEQ ID NO:8), VP22, a cell penetrating peptide from Herpes simplex virus, or a polyarginine peptide sequence.

In still other embodiments, the targeting endonuclease can comprise at least one marker domain. Non-limiting examples of marker domains include fluorescent proteins, purification tags, and epitope tags. In some embodiments, the marker domain can be a fluorescent protein. Non limiting examples of suitable fluorescent proteins include green fluorescent proteins (*e.g*., GFP, GFP-2, tagGFP, turboGFP, EGFP, Emerald, Azami Green, Monomeric Azami Green, CopGFP, AceGFP, ZsGreen1), yellow fluorescent proteins (*e.g*., YFP, EYFP, Citrine, Venus, YPet, PhiYFP, ZsYellow1), blue fluorescent proteins (*e.g*., BFP, EBFP, EBFP2, Azurite, mKalama1, GFPuv, Sapphire, T-sapphire), cyan fluorescent proteins (*e.g*., ECFP, Cerulean, CyPet, AmCyan1, Midoriishi-Cyan), red fluorescent proteins (*e.g*., mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), and orange fluorescent proteins (*e.g*., mOrange, mKO, Kusabira-Orange, Monomeric Kusabira-Orange, mTangerine, tdTomato) or any other suitable fluorescent protein. In other embodiments, the marker domain can be a purification tag and/or an epitope tag. Suitable tags include, but are not limited to, glutathione-S-transferase (GST), chitin binding protein (CBP), maltose binding protein, thioredoxin (TRX), poly(NANP), tandem affinity purification (TAP) tag, myc, AcV5, AU1, AU5, E, ECS, E2, FLAG, HA, nus, Softag 1, Softag 3, Strep, SBP, Glu-Glu, HSV, KT3, S, S1, T7, V5, VSV-G, 6xHis, biotin carboxyl carrier protein (BCCP), and calmodulin.

The one or more additional domains can be located at the N-terminus, the C-terminal, or in an internal location of the targeting endonuclease. Alternatively, the one or more additional domains can be fused directly or via a linker to the targeting endonuclease. Examples of suitable linkers are well known in the art and programs to design linkers are readily available (Crasto et al., Protein Eng., 2000, 13(5):309-312).

The targeting endonucleases described above can be expressed in and purified from eukaryotic or bacterial cells using techniques well-known in the art.

### (viii) Nucleic acids encoding targeting endonucleases

In some embodiments, the targeting endonuclease is introduced into the cell as a nucleic acid that encodes the targeting endonuclease. The nucleic acid encoding the targeting endonuclease can be DNA or RNA, linear or circular, single-stranded or double-stranded. The RNA or DNA can be codon optimized for efficient translation into protein in the eukaryotic cell of interest. Codon optimization programs are available as freeware or from commercial sources. In some embodiments, the nucleic acid encoding the targeting endonuclease can be mRNA. The mRNA encoding the targeting endonuclease can be transcribed *in vitro* and purified for introduction into the cell. The mRNA can be 5' capped and/or 3' polyadenylated. In other embodiments, the nucleic acid encoding the targeting endonuclease can be DNA. The DNA sequence encoding the targeting endonuclease can be operably linked to at least one promoter control sequence for expression in the cell of interest. In additional aspects, the DNA sequence encoding the targeting endonuclease also can be linked to a polyadenylation signal (e.g., SV40 polyA signal, bovine growth hormone (BGH) polyA signal, *etc.*) and/or at least one transcriptional termination sequence.

In some embodiments, the DNA coding sequence can be operably linked to a eukaryotic promoter sequence for expression in the eukaryotic cell of interest. The eukaryotic promoter control sequence can be constitutive, regulated, or cell- or tissue-specific. Suitable eukaryotic constitutive promoter control sequences include, but are not limited to, cytomegalovirus immediate early promoter (CMV), simian virus (SV40) promoter, adenovirus major late promoter, Rous sarcoma virus (RSV) promoter, mouse mammary tumor virus (MMTV) promoter, phosphoglycerate kinase (PGK) promoter, elongation factor (ED1)-alpha promoter, ubiquitin promoters, actin promoters, tubulin promoters, immunoglobulin promoters, fragments thereof, or combinations of any of the foregoing. Examples of suitable eukaryotic regulated promoter control sequences include without limit those regulated by heat shock, metals, steroids, antibiotics, or alcohol. Non-limiting examples of tissue-specific promoters include B29 promoter, CD14 promoter, CD43 promoter, CD45 promoter, CD68 promoter, desmin promoter, elastase-1 promoter, endoglin promoter, fibronectin promoter, Flt-1 promoter, GFAP promoter, GPIIb promoter, ICAM-2 promoter, INF-β promoter, Mb promoter, Nphsl promoter, OG-2 promoter, SP-B promoter, SYN1 promoter, and WASP promoter. The promoter sequence can be wild type or it can be modified for more efficient or efficacious expression.

In various embodiments, the DNA encoding the targeting endonuclease can be present in a DNA construct. Suitable constructs include plasmid vectors, phagemids, cosmids, artificial/mini-chromosomes, transposons, and viral vectors (e.g., lentiviral vectors, adeno-associated viral vectors, *etc.*)*.* In one embodiment, the DNA encoding the targeting endonuclease is present in a plasmid vector. Non-limiting examples of suitable plasmid vectors include pUC, pBR322, pET, pBluescript, and variants thereof. The vector can comprise additional expression control sequences (e.g., promoter sequence, enhancer sequence, Kozak sequence, polyadenylation sequence, transcriptional termination sequence, *etc.*)*,* selectable marker sequences (e.g., antibiotic resistance genes), origin of replication, and the like. Additional information can be found in "Current Protocols in Molecular Biology" Ausubel et al., John Wiley & Sons, New York, 2003 or "Molecular Cloning: A Laboratory Manual" Sambrook & Russell, Cold Spring Harbor Press, Cold Spring Harbor, NY, 3rd edition, 2001.

In embodiments in which the targeting endonuclease is a CRISPR/Cas protein or variant thereof, the expression vector comprising DNA sequence encoding the CRISPR/Cas protein or variant thereof can further comprise DNA sequence encoding one or more guide RNAs. The sequence encoding the guide RNA(s) generally is operably linked to at least one transcriptional control sequence for expression of the guide RNA(s) in the cell of interest. For example, DNA encoding the guide RNA(s) can be operably linked to a promoter sequence that is recognized by RNA polymerase III (Pol III). Examples of suitable Pol III promoters include, but are not limited to, mammalian U6, U3, H1, and 7SL RNA promoters.

### (d) Introducing into the cell

The method generally described herein comprises introducing into the cell (i) the targeting endonuclease or nucleic acid encoding the targeting endonuclease and (ii) the donor polynucleotide comprising the exogenous sequence. In embodiments in which the targeting endonuclease is a protein (i.e., ZFN, TALENS, meganucleases), the targeting endonuclease can be introduced into the cell as (i) a purified protein, (ii) encoding RNA or (iii) encoding DNA. In embodiments in which the targeting nuclease is a CRISPR/Cas system, the targeting endonuclease can be introduced into the cell as (i) a protein-guide RNA complex, (ii) a protein along with DNA encoding the guide RNA, (iii) RNA encoding the CRISPR/CAS nuclease along with DNA encoding the guide RNA, or (iv) DNA encoding both the nuclease and the guide RNA.

The targeting endonuclease molecule(s) and the donor polynucleotide can be introduced into the cell by a variety of means. Suitable delivery means include microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In specific embodiments, the targeting endonuclease molecule(s) and the donor polynucleotide can be introduced into the cell by nucleofection.

In embodiments in which more than one targeting endonuclease molecule and more than one donor polynucleotide are introduced into a cell, the molecules can be introduced simultaneously or sequentially. For example, targeting endonuclease molecules, each specific for a target site, and the donor polynucleotides can be introduced at the same time. Alternatively, each targeting endonuclease molecule and the donor polynucleotide can be introduced sequentially.

### (e) Culturing the cell

The method generally described herein further comprises maintaining the cell under appropriate conditions such that the exogenous sequence is integrated into the target site of the genomic sequence. In embodiments in which the exogenous sequence in the donor polynucleotide is flanked by sequences having substantial sequence identity to sequences flanking the target site in the genomic sequence, the targeting endonuclease introduces a double-stranded break at the target site in the genomic sequence, such that the exogenous sequence is integrated into the genomic sequence by a homology-directed process. In embodiments in which the exogenous sequence in the donor polynucleotide is flanked by sequences recognized by the targeting endonuclease, the targeting endonuclease introduces double-stranded breaks at the target site in the genomic sequence and at the recognition sequences flanking the exogenous sequence in the donor polynucleotide, such that the exogenous sequence is integrated into the genomic sequence by a direct ligation process.

In general, the cell is maintained under conditions appropriate for cell growth and/or maintenance. Suitable cell culture conditions are well known in the art and are described, for example, in Santiago et al. (2008) PNAS 105:5809-5814; Moehle et al. (2007) PNAS 104:3055-3060; Urnov et al. (2005) Nature 435:646-651; and Lombardo et al (2007) Nat. Biotechnology 25:1298-1306. Those of skill in the art appreciate that methods for culturing cells are known in the art and can and will vary depending on the cell type. Routine optimization may be used, in all cases, to determine the best techniques for a particular cell type.

Integration of the exogenous sequence can be confirmed by PCR (*e.g*., junction PCR), DNA sequencing, flow cytometry (*e.g*., when the exogenous sequence further comprises fluorescent protein coding sequence), selection techniques (*e.g*., when the exogenous sequence further comprises an antibiotic resistance gene), and other means well known in the art.

The exogenous sequence is stably integrated into the genome of the cell. In particular, the integrated sequence remains in the genomic locus and is not excised or altered in any manner. For example, the integrated sequence and/or adjacent sequences are not subject to gene silencing or position effects. Additionally, the integrated exogenous sequence does not affect the function of genes or other chromosomal sequences in the cell, *i.e*., global or local gene expression is not altered, there are no cell abnormalities or deficits, there is no position mutagenesis or other side effects, etc.. The integrated sequence is able to function predictably and reliably. For example, when the exogenous sequence encodes a protein or RNA molecule, expression of the exogenous sequence is stable, efficient, consistent, and predictable. Alternatively, when the exogenous sequence comprises one or more recognition sequences for a polynucleotide modification enzyme, the exogenous sequence can be used as a landing pad for subsequence integration of sequences of interest.

### (f) Types of cells

Generally suitable cells include mammalian cells or mammalian cell lines. Non-limiting examples of suitable mammalian cells include Chinese hamster ovary (CHO) cells; mouse myeloma NS0 cells; baby hamster kidney (BHK) cells; mouse embryonic fibroblast 3T3 cells (NIH3T3); mouse B lymphoma A20 cells; mouse melanoma B16 cells; mouse myoblast C2C12 cells; mouse myeloma SP2/0 cells; mouse embryonic mesenchymal C3H-10T1/2 cells; mouse carcinoma CT26 cells, mouse prostate DuCuP cells; mouse breast EMT6 cells; mouse hepatoma Hepa1c1c7 cells; mouse myeloma J5582 cells; mouse epithelial MTD-1A cells; mouse myocardial MyEnd cells; mouse renal RenCa cells; mouse pancreatic RIN-5F cells; mouse melanoma X64 cells; mouse lymphoma YAC-1 cells; rat glioblastoma 9L cells; rat B lymphoma RBL cells; rat neuroblastoma B35 cells; rat hepatoma cells (HTC); buffalo rat liver BRL 3A cells; canine kidney cells (MDCK); canine mammary (CMT) cells; rat osteosarcoma D17 cells; rat monocyte/macrophage DH82 cells; monkey kidney SV-40 transformed fibroblast (COS7) cells; monkey kidney CVI-76 cells; African green monkey kidney (VERO-76) cells; human embryonic kidney cells (HEK293, HEK293T); human cervical carcinoma cells (HELA); human lung cells (W138); human liver cells (Hep G2); human U2-OS osteosarcoma cells, human A549 cells, human A-431 cells, or human K562 cells. An extensive list of mammalian cell lines may be found in the American Type Culture Collection catalog (ATCC, Manassas, VA).

In various embodiments, the cell lines can be deficient in glutamine synthase (GS), dihydrofolate reductase (DHFR), hypoxanthine-guanine phosphoribosyltransferase (HPRT), or a combination thereof. For example, the chromosomal sequences encoding GS, DHFR, and/or HPRT can be inactivated. In specific embodiments, all chromosomal sequences encoding GS are inactivated in the cell lines.

In exemplary embodiments, the cells are Chinese Hamster Ovary (CHO) cells. Numerous CHO cell lines are available from American Type Culture Collection (ATCC). Suitable CHO cell lines include, but are not limited to, CHO-K1 cells and derivatives thereof. In some embodiments the CHO cell line can be CHOZN GS-/-, CHO-DXB11, CHO-DG44, CHO-S, or CHO-K1SV.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

When introducing elements of the present disclosure or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

As used herein, the term "endogenous sequence" refers to a chromosomal sequence that is native to the cell.

The term "exogenous sequence" refers to a chromosomal sequence that is not native to the cell, or a chromosomal sequence that is moved to a different chromosomal location.

A "genetically modified" cell refers to a cell in which the genome has been modified, *i.e.*, the cell contains at least chromosomal sequence that has been engineered to contain an insertion of at least one nucleotide, a deletion of at least one nucleotide, and/or a substitution of at least one nucleotide.

The terms "genome modification" and "genome editing" refer to processes by which a specific chromosomal sequence is changed such that the chromosomal sequence is modified. The chromosomal sequence may be modified to comprise an insertion of at least one nucleotide, a deletion of at least one nucleotide, and/or a substitution of at least one nucleotide. The modified chromosomal sequence is inactivated such that no product is made. Alternatively, the chromosomal sequence can be modified such that an altered product is made.

A "gene," as used herein, refers to a DNA region (including exons and introns) encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites, and locus control regions.

The term "heterologous" refers to an entity that is not native to the cell or species of interest.

The terms "nucleic acid" and "polynucleotide" refer to a deoxyribonucleotide or ribonucleotide polymer, in linear or circular conformation. For the purposes of the present disclosure, these terms are not to be construed as limiting with respect to the length of a polymer. The terms can encompass known analogs of natural nucleotides, as well as nucleotides that are modified in the base, sugar and/or phosphate moieties. In general, an analog of a particular nucleotide has the same base-pairing specificity; i.e., an analog of A will base-pair with T. The nucleotides of a nucleic acid or polynucleotide may be linked by phosphodiester, phosphothioate, phosphoramidite, phosphorodiamidate bonds, or combinations thereof..

The term "nucleotide" refers to deoxyribonucleotides or ribonucleotides. The nucleotides may be standard nucleotides (i.e., adenosine, guanosine, cytidine, thymidine, and uridine) or nucleotide analogs. A nucleotide analog refers to a nucleotide having a modified purine or pyrimidine base or a modified ribose moiety. A nucleotide analog may be a naturally occurring nucleotide (e.g., inosine) or a non-naturally occurring nucleotide. Non-limiting examples of modifications on the sugar or base moieties of a nucleotide include the addition (or removal) of acetyl groups, amino groups, carboxyl groups, carboxymethyl groups, hydroxyl groups, methyl groups, phosphoryl groups, and thiol groups, as well as the substitution of the carbon and nitrogen atoms of the bases with other atoms (e.g., 7-deaza purines). Nucleotide analogs also include dideoxy nucleotides, 2'-O-methyl nucleotides, locked nucleic acids (LNA), peptide nucleic acids (PNA), and morpholinos.

The terms "polypeptide" and "protein" are used interchangeably to refer to a polymer of amino acid residues.

As used herein, the terms "target site" or "target sequence" refer to a nucleic acid sequence that defines a portion of a chromosomal or genomic sequence to be modified or edited and to which a targeting endonuclease is engineered to recognize, bind, and cleave, provided sufficient conditions for binding and cleavage exist.

The terms "upstream" and "downstream" refer to locations in a nucleic acid sequence relative to a fixed position. Upstream refers to the region that is 5' (i.e., near the 5' end of the strand) to the position and downstream refers to the region that is 3' (i.e., near the 3' end of the strand) to the position.

Techniques for determining nucleic acid and amino acid sequence identity are known in the art. Typically, such techniques include determining the nucleotide sequence of the mRNA for a gene and/or determining the amino acid sequence encoded thereby, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, Wis.) in the "BestFit" utility application. Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+Swiss protein+Spupdate+PIR. Details of these programs can be found on the GenBank website. With respect to sequences described herein, the range of desired degrees of sequence identity is approximately 80% to 100% and any integer value therebetween. Typically the percent identities between sequences are at least 70-75%, preferably 80-82%, more preferably 85-90%, even more preferably 92%, still more preferably 95%, and most preferably 98% sequence identity.

### EXAMPLES

The following examples illustrate certain aspects of the invention.

### Example 1: Identification of Genomic Loci of Randomly Integrated Transgenes in CHO Cell Lines

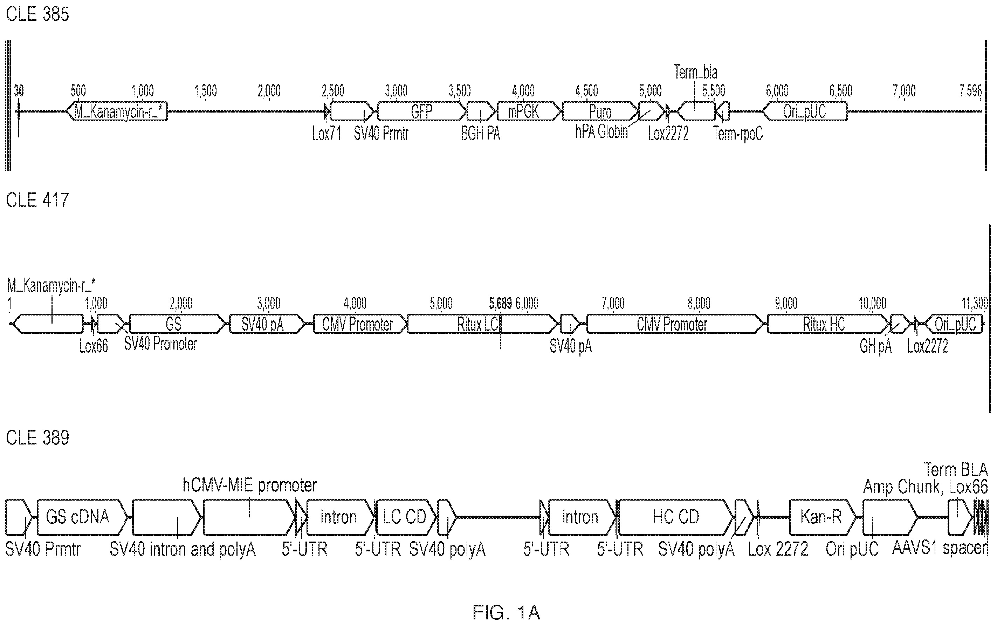

Approach 1. Random integration of a plasmid, CLE 385 (see FIG. 1A), containing a GFP expressing cassette landing pad with flanking Lox sites Lox71 and Lox2272. High GFP expressing single copy clones were identified and characterized. The genomic sequences flanking the integration events were then Blasted against available CHO databases to best determine the contig Accession number and location in the contig of the randomly integrated landing pad.

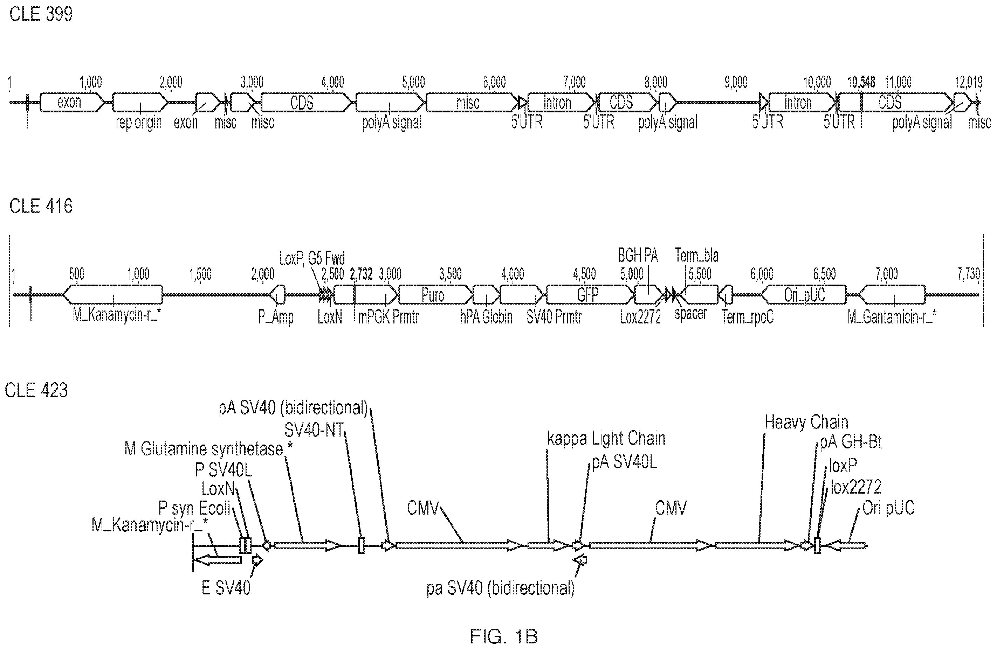

Approach 2. Random integration of a plasmid, CLE 399 (See FIG. 1B), containing a monoclonal antibody expressing cassette landing pad with flanking Lox sites LoxP and LoxN on the 5' and Lox2272 on the 3'. High mAb expressing single copy clones were identified and characterized. Recombinase mediated cassette exchange (RMCE) was then used to exchange a GFP expressing cassette, CLE 416 (See FIG. 1B), and high GFP expressing clones were identified and characterized. The genomic sequences flanking the integration events were then Blasted against available CHO databases to best determine the contig Accession number and location in the contig of the randomly integrated landing pad.

The results are shown in Table 1:

| Table 1. Genomic Location of Randomly Integrated Transgenes | | |
|---|---|---|
| **Reference ID** | **Insertion Site (Scaffold:Range)** | **Locus Name** |
| NW_003613934.1 | Scaffold 979: 1,090,000 - 1,127,000 | D145 |
| NW_003613732.1 | Scaffold 1721: 1,935,000 - 1,985,000 | MP 7 |
| NW_003614159.1 | Scaffold 3466: 365,000 - 395,000 | MP 58 |

### Example 2. Validation of Genomic Locus D145

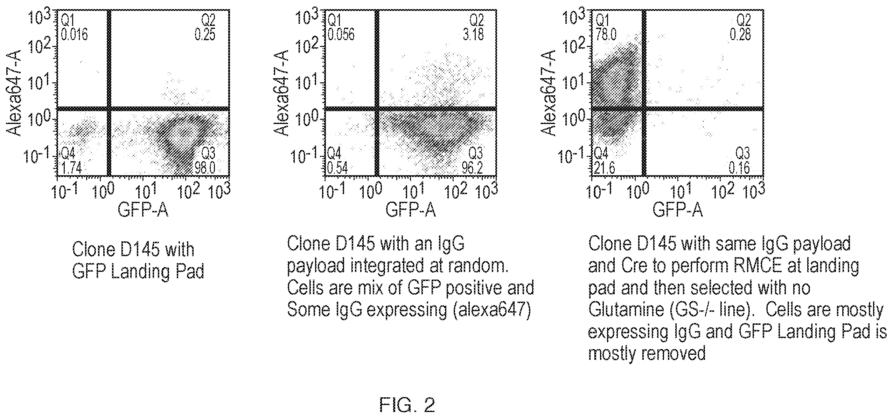

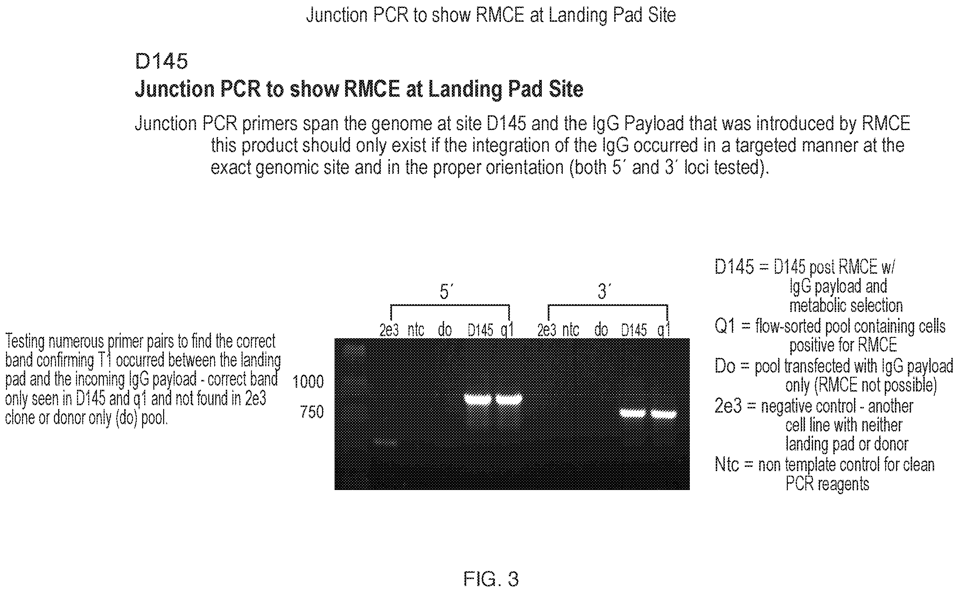

A clone with the landing pad CLE 385 (clone D145) was isolated. Recombinase mediated cassette exchange (RMCE) was performed on this clone. Briefly, a plasmid donor (CLE 389, FIG. 1A), which contains mAb cassette flanked by Lox sites compatible with the landing pad CLE 385, was introduced into the clone by electroporation along with Cre mRNA. After successful RMCE, the CLE 385 landing pad would be replaced by the donor cassette in CLE 389. After glutamine selection, the resulting pool was analyzed by flow cytometry (FIG. 2) and compared to the D145 parental clone and a pool which was generated using only the donor without Cre mRNA. Compared to the donor-only pool, the majority of cells in the RMCE pool had shifted from GFP expression to mAb expression. PCR primers were designed such that an amplification event could only occur of the mAb expression cassette had inserted at the D145 locus specifically and correctly. Amplification was successful at both the 5 prime and 3 prime junctions (FIG. 3).

### Example 3. Validation of Genomic Locus MP 7 (reference example)

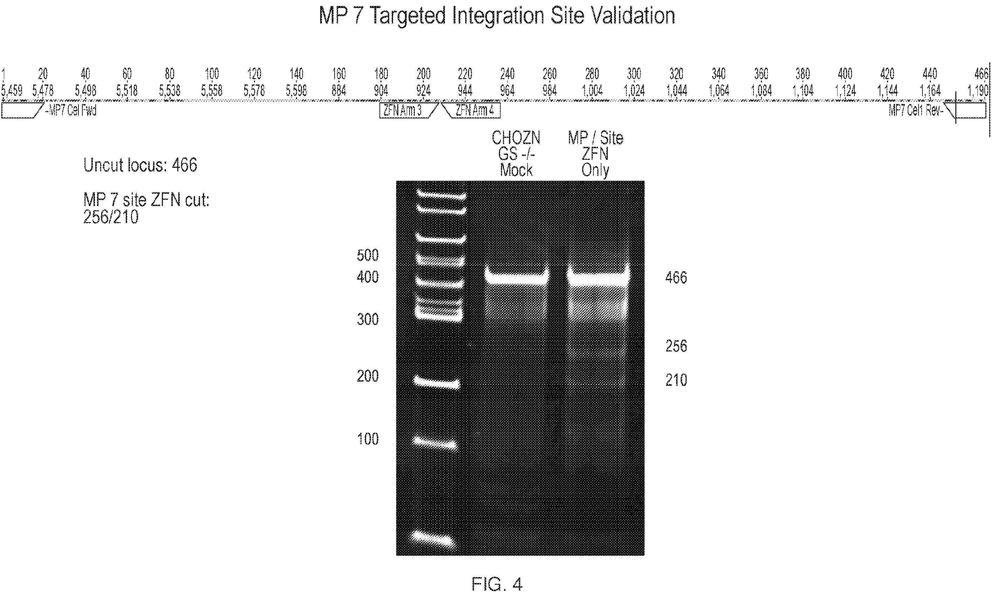

Site MP 7 was identified by approach 2 and retargeted with CLE 416 to create a pool containing cells with a GFP landing pad. This pool was then targeted with a mAb expression plasmid containing matching Lox sites and tested for antibody secretion. Efforts to retarget MP 7 with ZFNs and a new landing pad are underway (FIG. 4).

### Example 4. Validation of Genomic Locus MP 58 (reference example)

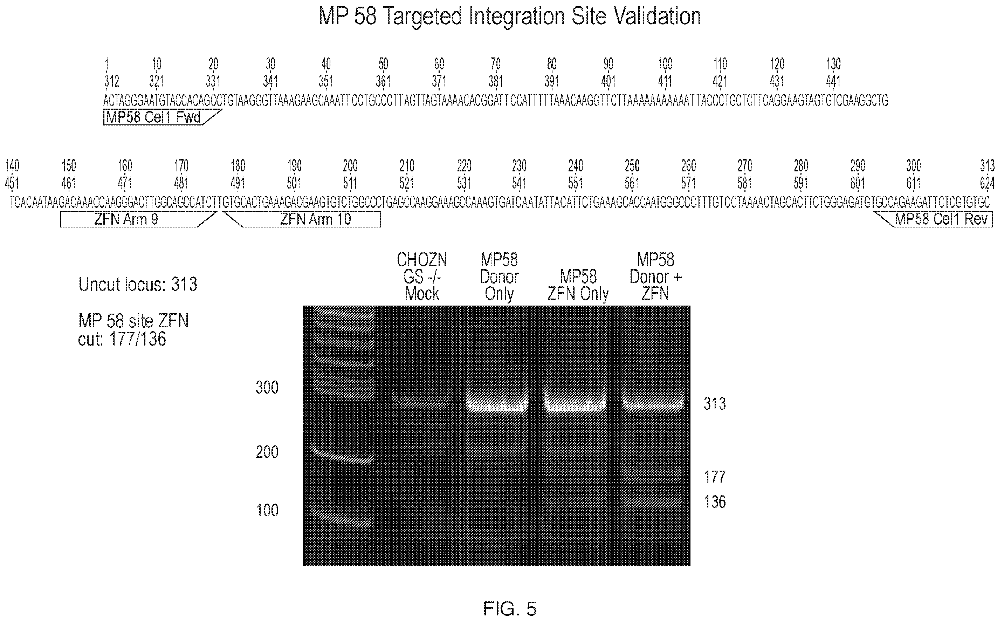

Site MP 58 was identified by approach 2 and retargeted with CLE 416 to create a pool containing cells with a GFP landing pad. ZFNs were designed for the purpose of placing a new landing pad at the site (FIG. 5).

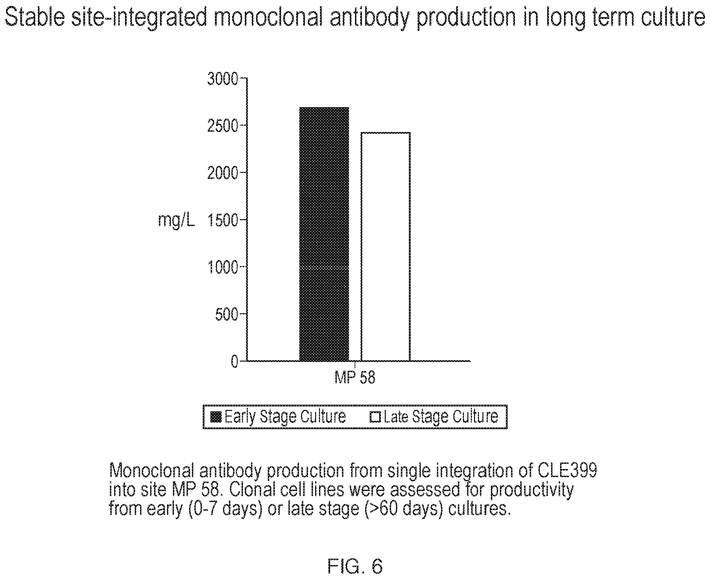

Monoclonal antibody production generated from Site MP 58 was measured in early and late stage cultures by standard growth and productivity analysis. Briefly, 30 ml cell cultures were begun with an initial seeding density of 3x105 cells/ml in 50 ml shake tubes and supplemented on a regular schedule with standard feeding conditions. Growth and viability was monitored as cell cultures were harvested and analyzed for secreted monoclonal antibody concentrations (ForteBio Octet, FIG. 6).

## Claims

1. A method for stable integration of at least one exogenous sequence into genomic DNA of a Chinese hamster ovary (CHO) cell, the method comprises integrating the at least one exogenous sequence into a site within the region bp 1,090,000 to bp 1,127,00 of genomic sequence NCBI Reference Sequence NW_003613934.1.

2. The method of claim 1, wherein the at least one exogenous sequence encodes a protein or an RNA molecule.

3. The method of claim 2, wherein the protein is a therapeutic protein, a recombinant protein, or an industrial protein.

4. The method of claim 2, wherein the RNA molecule is a small interfering RNA (siRNA), a micro RNA (miRNA), a guide RNA (gRNA), or a precursor thereof.

5. The method of any one of claims 2 to 4, wherein the at least one exogenous sequence is operably linked to a promoter control sequence.

6. The method of any one of claims 2 to 5, wherein expression of the exogenous sequence is stable, predictable, and reproducible.

7. The method of claim 1, wherein the at least one exogenous sequence comprises at least one recognition sequence for a polynucleotide modification enzyme.

8. The method of claim 7, wherein the at least one recognition sequence comprises a nucleic acid sequence that does not exist endogenously in the genome of the mammalian cell.

9. The method of claim 7, wherein the polynucleotide modification enzyme is a site-specific recombinase or a targeting endonuclease.

10. The method of claim 9, wherein the site-specific recombinase is Bxb1 integrase, Cre recombinase, FLP recombinase, gamma delta resolvase, lambda integrase, phi C31 integrase, R4 integrase, Tn3 resolvase, or TP901-1 recombinase.

11. The method of claim 9, wherein the targeting endonuclease is a zinc finger nuclease (ZFN), a clustered regularly interspersed short palindromic repeats (CRISPR)/CRISPR-associated (Cas) (CRISPR/Cas) nuclease system, a CRISPR/Cas dual nickase system, a transcription activator-like effector nuclease (TALEN), a meganuclease, or a fusion protein comprising a programmable DNA-binding domain and a nuclease domain.

## Patentansprüche

1. Verfahren zur stabilen Integration von mindestens einer exogenen Sequenz in die genomische DNA einer CHO-Zelle (Chinese Hamster Ovary), wobei das Verfahren Integrieren der mindestens einen exogenen Sequenz in eine Stelle innerhalb der Region bp 1.090.000 bis bp 1.127.000 der genomischen Sequenz der NCBI-Referenzsequenz NW_003613934.1 umfasst.

2. Verfahren nach Anspruch 1, wobei die mindestens eine exogene Sequenz ein Protein oder ein RNA-Molekül codiert.

3. Verfahren nach Anspruch 2, wobei es sich bei dem Protein um ein therapeutisches Protein, ein rekombinantes Protein oder ein industrielles Protein handelt.

4. Verfahren nach Anspruch 2, wobei es sich bei dem RNA-Molekül um eine small interfering RNA (siRNA), eine Mikro-RNA (miRNA), eine Guide-RNA (gRNA) oder eine Vorstufe davon handelt.

5. Verfahren nach einem der Ansprüche 2 bis 4, wobei die mindestens eine exogene Sequenz mit einer Promotor-Steuersequenz wirkverknüpft ist.

6. Verfahren nach einem der Ansprüche 2 bis 5, wobei eine Expression der exogenen Sequenz stabil, vorhersagbar und reproduzierbar ist.

7. Verfahren nach Anspruch 1, wobei die mindestens eine exogene Sequenz mindestens eine Erkennungssequenz für ein Polynukleotid-Modifikationsenzym umfasst.

8. Verfahren nach Anspruch 7, wobei die mindestens eine Erkennungssequenz eine Nukleinsäuresequenz umfasst, die nicht endogen in dem Genom der Säugetierzelle existiert.

9. Verfahren nach Anspruch 7, wobei es sich bei dem Polynukleotid-Modifikationsenzym um eine ortsspezifische Rekombinase oder eine zielgerichtete Endonuklease handelt.

10. Verfahren nach Anspruch 9, wobei es sich bei der ortsspezifischen Rekombinase um Bxb1-Integrase, Cre-Rekombinase, FLP-Rekombinase, Gamma-Delta-Resolvase, Lambda-Integrase, Phi-C31-Integrase, R4-Integrase, Tn3-Resolvase oder TP9011-Rekombinase handelt.

11. Verfahren nach Anspruch 9, wobei es sich bei der zielgerichteten Endonuklease um eine Zinkfingernuklease (ZFN), ein Clustered Regularly Interspersed Short Palindromic Repeats (CRISPR)/CRISPR-assoziiertes (Cas) (CRISPR/Cas) Nukleasesystem, ein CRISPR/Cas-Dual-Nickase-System, eine transkriptionsaktivatorartige Effektornuklease (TALEN), eine Meganuklease oder ein Fusionsprotein, umfassend eine programmierbare DNA-Bindungsdomäne und eine Nukleasedomäne, handelt.

## Revendications

1. Procédé d'intégration stable d'au moins une séquence exogène dans l'ADN génomique d'une cellule d'ovaire de hamster chinois (CHO), le procédé comprend l'intégration de l'au moins une séquence exogène dans un site situé dans la région paire de bases 1 090 000 à paire de bases 1 127 000 de la séquence génomique NCBI Reference Sequence NW_003613934.1.

2. Procédé selon la revendication 1, dans lequel l'au moins une séquence exogène code une protéine ou une molécule d'ARN.

3. Procédé selon la revendication 2, dans lequel la protéine est une protéine thérapeutique, une protéine recombinante ou une protéine industrielle.

4. Procédé selon la revendication 2, dans lequel la molécule d'ARN est un petit ARN interférent (siARN), un micro-ARN (miARN), un ARN guide (gARN) ou un précurseur de ceux-ci.

5. Procédé selon l'une quelconque des revendications 2 à 4, dans lequel l'au moins une séquence exogène est liée de manière fonctionnelle à une séquence de contrôle de promoteur.

6. Procédé selon l'une quelconque des revendications 2 à 5, dans lequel l'expression de la séquence exogène est stable, prévisible et reproductible.

7. Procédé selon la revendication 1, dans lequel l'au moins une séquence exogène comprend au moins une séquence de reconnaissance pour une enzyme de modification de polynucléotides.

8. Procédé selon la revendication 7, dans lequel l'au moins une séquence de reconnaissance comprend une séquence d'acide nucléique qui n'existe pas de manière endogène dans le génome de la cellule de mammifère.

9. Procédé selon la revendication 7, dans lequel l'enzyme de modification de polynucléotides est une recombinase spécifique du site ou une endonucléase de ciblage.

10. Procédé selon la revendication 9, dans lequel la recombinase spécifique du site est l'intégrase Bxb1, la recombinase Cre, la recombinase FLP, la résolvase gamma delta, l'intégrase lambda, l'intégrase phi C31, l'intégrase R4, la résolvase Tn3 ou la recombinase TP9011.

11. Procédé selon la revendication 9, dans lequel l'endonucléase de ciblage est une nucléase à doigt de zinc (ZFN), un système de nucléase courtes répétitions palindromiques regroupées et régulièrement espacées (CRISPR)/CRISPR et protéines associées à (Cas) (CRISPR/Cas), un système double nickase CRISPR/Cas, une nucléase effectrice de type activateur de transcription (TALEN), une méganucléase, ou une protéine de fusion comprenant un domaine de liaison à l'ADN programmable et un domaine nucléase.
